# FASCICULE DE BREVET EUROPEEN

(11) **EP 0 571 619 B1**
(45) Date de publication et mention de la délivrance du brevet: **09.04.1997**
(21) Numéro de dépôt: 93902307.3
(22) Date de dépôt: 08.12.1992
(51) Int. Cl.: A61B 17/58, A61B 17/70

(54) **DISPOSITIFS DE REDRESSEMENT, FIXATION, COMPRESSION, ELONGATION DU RACHIS**
VORRICHTUNGEN ZUM GERADERICHTEN, BEFESTIGEN, ZUSAMMENDRÜCKEN UND DEHNEN DER WIRBELSÄULE
DEVICES FOR STRAIGHTENING, CLAMPING, COMPRESSING AND STRETCHING THE SPINE

(30) Priorité: 12.12.1991 FR 9115453
(43) Date de publication de la demande: 01.12.1993
(73) Titulaire: JBS, Société Anonyme, F-10000 Troyes (FR)
(72) Inventeur: JEANSON, Jean-François, F-10000 Troyes (FR); MARNAY, Thierry, F-30900 Nimes (FR)
(74) Mandataire: Gérardin, Robert Jean René
(86) Numéro de dépôt international: FR9201159
(87) Numéro de publication internationale: WO9311715

(56) Documents cités:
- EP-A- 0 410 309
- EP-A- 0 443 892
- EP-A- 0 452 792
- WO-A-91/06261
- DE-A- 3 538 593
- DE-B- 2 649 042
- DE-U- 9 006 694
- FR-A- 2 650 173
- GB-A- 2 096 465
- GB-A- 2 198 647
- US-A- 2 847 014
- US-A- 4 347 845
- US-A- 4 633 862
- US-A- 5 020 519

## Description

L'invention concerne les dispositifs de redressement, de fixation, de compression et d'élongation de rachis, présentant une déviation anormale, comportant des implants vissés et des crochets reliés par des tiges, ou barres, auxquels elles se solidarisent par blocage dans des rainures par l'intermédiaire d'un système à vis et écrou, ainsi qu'à leur instrumentation de mise en oeuvre.

On connaît déjà, par la demande de brevet français FR-A-2 650 173, un dispositif sur lequel la tête des implants et des crochets comporte des rainures longitudinales, dans le fond desquelles se bloquent les tiges de liaison et d'étaiement du rachis. Ce blocage résulte de la déformation, par rapprochement, des côtés de la rainure, par l'intermédiaire d'un système à vis cylindrique, réalisé autour de la tête des dits implants et crochets, et d'un écrou conique, qui, lors de son vissage, assure le rapprochement progressif des côtés de la rainure et, par conséquent, un sertissage partiel de la tige dans le fond des rainures, et le serrage de la tige contre le fond de celles-ci, par la base de l'écrou, qui a été crantée pour s'opposer au dévissage intempestif.

Pour parfaire la solidarisation de l'ensemble, les tiges ont été striées à leur périphérie, ainsi que le fond de la rainure de la tête des implants vissés et des crochets.

La liaison longitudinale et latérale des tiges, ou barres, est obtenue par l'intermédiaire de deux têtes d'implants, dépourvues de leur vis ou crochet d'implantation, et d'une plaquette moletée, comportant deux orifices permettant le passage des têtes d'implants, dont l'un est circulaire, alors que l'autre est oblong pour offrir une latitude de réglage.

Le système de compression ou de distraction de deux vertèbres, l'une par rapport à l'autre, peut être constitué de deux crochets, à tête rainurée et filetée extérieurement, en tout point analogue à celle des implants vissés, se fixant sur les tiges par le même moyen, mais reliés, en plus, par une tige filetée, destinée à permettre la compression des vertèbres par rapprochement des crochets, avant solidarisation définitive de ceux-ci à la tige.

L'instrumentation de mise en oeuvre se limite aux outillages courants, à une pince spéciale permettant la préhension et la présentation des crochets, à un porte-écrou permettant la mise en place des écrous sur la tête des vis et à un pousse-barre destiné à forcer les barres, ou tiges, à pénétrer dans la tête des implants à vis et à maintenir les dites barres, ou tiges, dans le fond des dites rainures, pendant que l'on visse l'écrou de serrage.

La présente invention telle qu'elle est caractérisée dans les revendications a pour but de doter les dispositifs de redressement, de fixation, de compression et d'élongation du rachis, de conception correspondante, de moyens permettant de renforcer la solidité de l'ensemble et de faciliter sa mise en oeuvre.

Les avantages obtenus, grâce à cette invention, consistent, essentiellement, en ce que l'adaptation du dispositif à toutes les dimensions de tiges, ou de barres, est aisée, tout en garantissant un maximum de rigidité et de fiabilité à l'ensemble de la prothèse ; ceci en évitant les possibilités d'erreur, ainsi que les tâtonnements préjudiciables à une intervention chirurgicale délicate.

D'autres caractéristiques et avantages apparaîtront dans la description qui va suivre d'un mode de réalisation du dispositif selon l'invention, donné à titre d'exemple non limitatif, au regard des dessins annexés, sur lesquels :
- la figure 1 représente une tête d'implants à vis, à arêtes de la mortaise chanfreinées,
- la figure 2 représente la vue de face d'une tête d'implants à vis, équipée d'un adaptateur pour tiges de diamètre inférieur à la largeur de la mortaise,
- la figure 3 représente la vue de côté, en coupe longitudinale, d'une tête d'implants à vis, équipée d'un adaptateur pour tiges de diamètre inférieur à la largeur de la mortaise,
- la figure 4 représente une vue de face d'une tête d'implants à vis, équipée d'une agrafe d'immobilisation en rotation,
- la figure 5 représente une vue de côté, en coupe longitudinale, d'un implant à vis, équipé de l'agrafe, avec représentation de la tige, ou barre, et de la surface de la vertèbre en trait mixte,
- la figure 6 représente une vue de côté, en perspective, d'un connecteur de tiges, ou barres, avec indication, en trait mixte, de l'extrémité de chacun des éléments de tiges, ou de barres, connecté,
- la figure 7 représente une vue de côté, en coupe partielle, d'une pince pédiculaire et laminaire,
- la figure 8 représente une vue de face d'une pince laminaire,
- la figure 9 représente une vue en coupe, selon AA, indiquée sur la figure 7, d'une pince pédiculaire,
- la figure 10 représente une vue en coupe, selon AA, indiquée sur la figure 7, d'une pince laminaire,
- la figure 11 représente une vue en élévation de la vis assurant la liaison des crochets de la pince pédiculaire et laminaire,
- la figure 12 représente une vue en coupe longitudinale partielle de la tête de la vis représentée à la figure 11,
- la figure 13 représente une demi-vue de dessus de la tête de la vis représentée à la figure 11,
- la figure 14 représente une vue de face d'un cavalier de liaison latérale de deux tiges, ou barres, avec représentation, en trait mixte, de l'extrémité correspondante de la plaquette de liaison,
- la figure 15 représente une vue de dessus d'un cavalier de liaison latérale de deux tiges, ou barres,
- la figure 16 représente une vue de côté du pousse-tiges, ou barres, en place sur une tête d'implants représentée en trait mixte,
- la figure 17 représente une vue de face partielle de l'extrémité de la glissière, en place dans les gorges de l'embase d'un crochet à tête rainurée et sur une tige, représentée en trait mixte,
- la figure 18 représente une vue de dessus, en perspective et en coupe partielle, selon BB, indiquée sur la figure 16, des extrémités de la glissière et du coulisseau, avec représentation de la tige en trait mixte,
- la figure 19 représente une vue de face partielle d'une pince anti-rotation, en position de serrage, avec représentation de la tige, ou barre, en trait mixte,
- la figure 20 représente une vue de côté partielle d'une pince anti-rotation, en position de serrage,
- la figure 21 représente une vue de face d'une pince étau porte-tiges, ou barres, en position de serrage,
- la figure 22 représente une vue de côté d'une pince étau porte-tiges, ou barres,
- la figure 23 représente une vue de face partielle des mors de serrage de la pince étau, selon les figures 21 et 22,
- la figure 24 représente une vue de côté, en coupe partielle, d'un porte-écrou,
- la figure 25 représente une vue de côté, en demi-coupe, d'un porte-implants à vis et crochets, à tête rainurée, pour vertèbres cervicales,

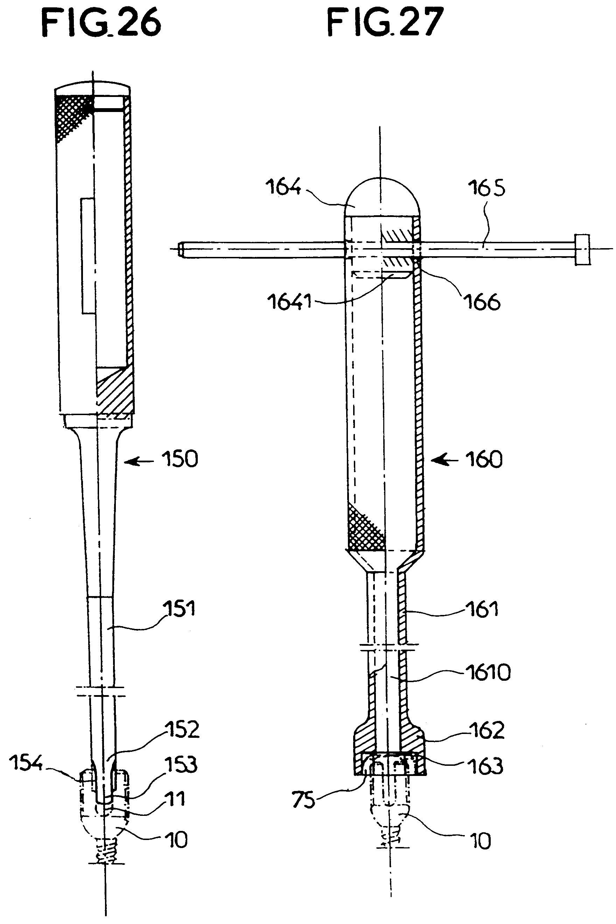

- la figure 26 représente une vue en élévation, en coupe partielle, d'un tournevis à ailettes d'entraînement,
- la figure 27 représente une vue en élévation, en coupe partielle, d'une clé de serrage de l'écrou de blocage des tiges de liaison dans la rainure de la tête des implants à vis et des crochets,

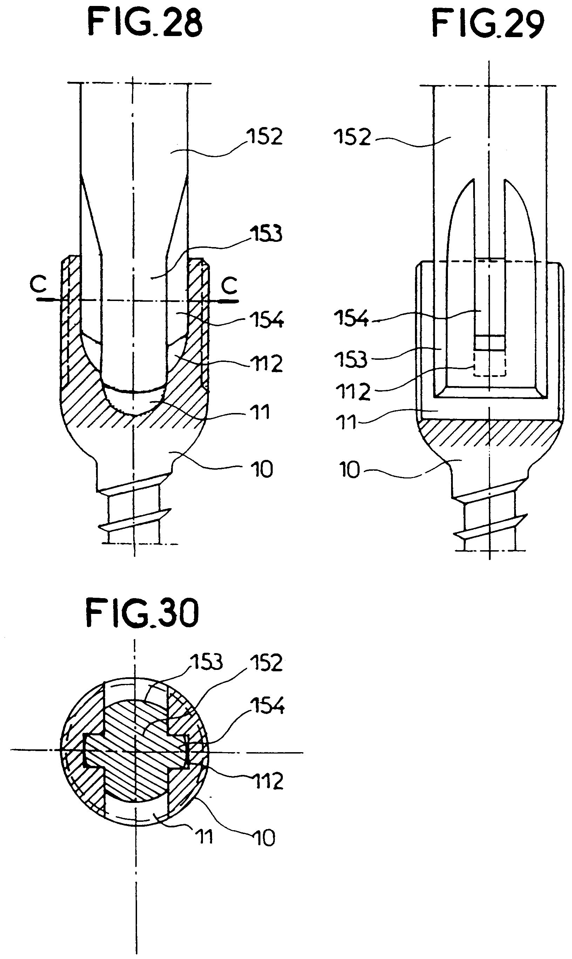

- la figure 28 représente une vue de face, en coupe partielle, d'une tête d'implants à vis, en cours de vissage par l'intermédiaire du tournevis représenté à la figure 26,
- la figure 29 représente une vue de côté, en coupe partielle, d'une tête d'implants à vis, en cours de vissage par l'intermédiaire du tournevis représenté à la figure 26,
- la figure 30 représente une vue de dessus, en coupe selon CC, portée sur la figure 28, d'une tête d'implants à vis, en cours de vissage par l'intermédiaire du tournevis représenté à la figure 26,
- la figure 31 représente une vue en élévation, en coupe partielle, d'une clé de contre-blocage, en place sur une tige en cours de blocage par un écrou manoeuvré par l'intermédiaire de la clé représentée à la figure 27,
- la figure 32 représente une vue de dessous partielle de la clé de contre-blocage, en place sur une tige en cours de blocage par un écrou,
- la figure 33 représente une vue en élévation, en coupe partielle, de la combinaison d'une clé de serrage de I'écrou de blocage des tiges de liaison, représentée figure 27, avec le tournevis représenté figure 26, en place sur une tête d'implants munie de son écrou,
- la figure 34 représente une vue en élévation d'un ciseau de préparation de l'accès, pour mise en place des crochets pédiculaires,
- la figure 35 représente une vue de côté partielle de l'extrémité du ciseau de préparation selon la figure 34,
- la figure 36 représente une vue de côté, en coupe partielle, d'une pince auto-stable pour vertèbres cervicales,
- la figure 37 représente une vue de côté d'une pince de compression, destinée à rapprocher deux implants,
- la figure 38 représente une vue de face d'une pince de compression, destinée à rapprocher deux implants, selon la figure 37,
- la figure 39 représente une vue de face d'une pince de distraction coudée, destinée à écarter deux implants,
- la figure 40 représente une vue de côté partielle des becs de la pince à distraction coudée, destinée à écarter deux implants, selon la figure 39,
- la figure 41 représente une vue de face dune pince pousse-tiges d'étaiement,
- la figure 42 représente une vue de côté de la pince selon la figure 41,
- la figure 43 représente une tête d'implant munie d'une cale de compensation,
- la figure 44 représente une vue de dessous de la cale selon la figure 43 ci-dessus,
- la figure 45 représente une vue de dessus d'une plaquette de liaison à deux orifices de passage oblong.
- la figure 46 représente une vue de côté de la plaquette de liaison selon la figure 45,
- la figure 47 représente une vue de dessus d'une plaquette de liaison à orifice circulaire et oblong,
- la figure 48 représente une vue de côté de la plaquette de liaison selon la figure 47,

Les figures représentent le dispositif de redressement, de fixation, de compression et d'élongation du rachis selon l'invention, comportant des têtes d'implants à vis **10**, à rainure longitudinale **11** à chanfrein **111** et à rainure transversale **112**, supportant une tige moletée **20** ou **21**, directement ou par l'intermédiaire d'un adaptateur **30** à talons **31**, **32**, ou d'une agrafe **40** à parties convexe **41** et concave **42** et à extrémités coudées et effilées **43**, **44** ; les éléments de tiges **20a** et **20b** étant reliés, longitudinalement, par des connecteurs **50**, constitués de deux têtes **51** et **52** à rainures **511** et **521** et à écrous **53** et **54**, solidarisés par une barrette **55**, de façon que les rainures soient disposées' parallèlement entre elles et obliquement, selon un angle alpha, par rapport au plan médian longitudinal (MM) de la barrette, de façon à obtenir un certain cheyauchement latéral des extrémités des éléments de tiges **20a** et **20b** ; les dits éléments de tiges **20a** et **20b** étant reliés transversalement aux autres éléments de tiges latéraux par l'intermédiaire de cavaliers **60** à embase **61** à gorges **611**, à tête filetée **62**, à rainure **63** et à écrou de blocage **64** reliés par une plaquette de liaison **65** à deux orifices de passage oblong **651** ou une plaquette de liaison **66** à un orifice **661** et une rainure oblongue **662**, et fixés aux pédicules et aux lames des vertèbres par des pinces pédiculaires et laminaires **70**, comportant un crochet **71** ou **72** à embase carrée **710** ou **720**, à gorges de préhension **711** ou **721**, et à tête **73** ou **74** rainurée **730** ou **740**, dans le fond de laquelle est maintenue, par un écrou **75**, la tige **20**, et un crochet simple **76**, relié à l'autre crochet **71** ou **72** par une vis cylindrique à tête fraisée **77**, à empreinte d'entraînement polygonale **771** et à embout d'alignement **772**, venant se visser dans l'orifice fileté **761** à ouverture conique **762**, percé dans l'embase du dit crochet simple **76**, après passage par l'un des deux orifices lisses **712** et **713** ou **722** et **723**, à entrée fraisée, réalisés dans l'embase **710** ou **720** du crochet **71** ou **72** ; les tiges, ou barres, **20**, **21** étant poussées à fond de rainure **11** des têtes **10** des implants à vis ou des têtes de certains crochets **71**, **72**, ou cavaliers **60**, par l'intermédiaire d'un pousse-tiges, ou barres, **80** à glissière **81** à fourche transversale **810** et à coulisseau **82** à fourche longitudinale **820**, avec déplacement relatif par l'intermédiaire d'une vis **83** à bouton moleté **84** ; ou d'une pince pousse-tiges ou barres **85**, constituée de deux leviers **851** et **852** à becs incurvés et striés, articulés transversalement chacun par rapport aux branches **853** et **854** d'une pince dont les becs **855** et **856** épousent la forme de la base de la tête des implants à vis **10** ; les tiges, ou barres, **20**, **21** étant manutentionnées et mises en place à l'aide d'une pince anti-rotation **90**, à becs fourchus **91 et 92** rappelés par des branches **921 et 911** articulées, maintenus, en position de serrage, par une crémaillère **93** à gâchette **931** rappelée par un ressort **94** vers un ergot **912** ; puis, après déformation à la demande du rachis du patient, tournées d'un demi-tour à l'aide de la pince étau **100**, à becs **101** et **102**, à empreintes en V **103** et **104**, striées transversalement, rappelés l'un vers l'autre par des branches **105** et **106**, avec maintien, en position de serrage, par une crémaillère **108** à rappel par un ressort **107** vers un ergot **109** ; les écrous **75** étant présentés à l'aide d'un porte-écrou **120**, à tige moletée **121** et à embout mâle **122** à embase **123** ; les vis et crochets à tête filetée et à rainure étant présentés à l'aide d'un porte-vis et crochets **130**, à manche tubulaire moleté **131** et à tige **132** munie, à son extrémité, d'un embout femelle **133**, aux dimensions des têtes filetées correspondantes ; les vertèbres cervicales étant, après dégagement à l'aide d'un ciseau **146**, reliées et corrigées par l'intermédiaire de crochets **140**, **143**, reliés par une vis **144** ; les têtes filetées des implants à vis **10**, des crochets **71**, **140** et des cavaliers **60** pouvant être, selon le cas, entraînées ou immobilisées en rotation à l'aide d'un tournevis **150** à tige **151**, à embout **152**, à ailettes perpendiculaires **153 et 154** pénétrant, respectivement, dans la rainure longitudinale et dans la rainure transversale de la dite tête filetée ; le dit tournevis **150** pouvant être utilisé en combinaison avec une clé tubulaire **160**, à pommeau amovible **164**, retenu à l'extrémité du manche par un embout **1641**, et une tige de manoeuvre **165**, passant par des orifices transversaux **166** réalisés dans le manche ; la dite tige **151** du tournevis **150** ayant une longueur et un diamètre correspondant à ceux de la tige **161** et de l'âme **1610** de cette dernière, de façon à permettre un centrage précis de l'écrou **75** de blocage de la tige **20**, **21** par rapport à la tête filetée et rainurée des implants à vis **10**, des crochets **71**, **140** et des cavaliers **60**, puis une immobilisation en rotation de la dite tête, pendant l'engagement de l'écrou **53**, **54**, **64**, **75** de sertissage et de blocage de la tige, par engagement de l'écrou dans l'empreinte **163** de l'embout **162** de la clé, puis de la tige **151** du tournevis **150**, successivement dans l'orifice fileté de l'écrou, puis dans les rainures longitudinale et transversale des têtes filetées, par son extrémité **152**, avant entraînement, en rotation, de l'écrou par l'intermédiaire de la clé **160** dépourvue de son pommeau **164** et de sa tige de manoeuvre **165** ; l'écrou étant, ensuite, approché de la tige **20**, **21** par l'intermédiaire de la clé tubulaire **160**, après retrait du tournevis **150** et remontage du pommeau **164** et de la tige de manoeuvre **165** ; les implants ayant été, si nécessaire, rapprochés à laide d'une pince de compression **180**, à branches **181 et 182**, à butée d'ouverture **186**, à becs **183 et 184**, a échancrure **185** au diamètre de l'embase de la vis des implants **10**, avec blocage en position par une crémaillère escamotable **187**, ou écartés à l'aide d'une pince de distraction **190**, à branches **191 et 192**, à becs **193 et 194**, à échancrure **195** au diamètre de l'embase de la vis des implants **10**, et à crémaillère de blocage **197** ; les branches **181**, **182 et 191**, **192** des dites pinces étant contrecoudées latéralement, selon un angle bêta de 45°, pour permettre le passage et la manoeuvre de la clé de serrage **160** ; ces pinces étant démontées après solidarisation des têtes filetées avec les tiges par un premier serrage, puis remplacées par une clé de contre-blocage **170**, à manche **171** fixé sur une tige **172**, dont l'autre extrémité est munie d'une fourche **173**, inclinée selon un angle alpha de 135°, par rapport à la tige **172**, dont l'extrémité des dents **174 et 175** comporte une échancrure semi-circulaire **176**, de dimensions correspondant à la section de la tige **20**, **21**, destinée à s'engager sur la dite tige, de chaque côté de la tête **10** de l'implant, en laissant un passage suffisant pour permettre le passage et la rotation de l'embout **162** de la clé de serrage pendant l'opération de blocage définitif de l'écrou sur la tige.

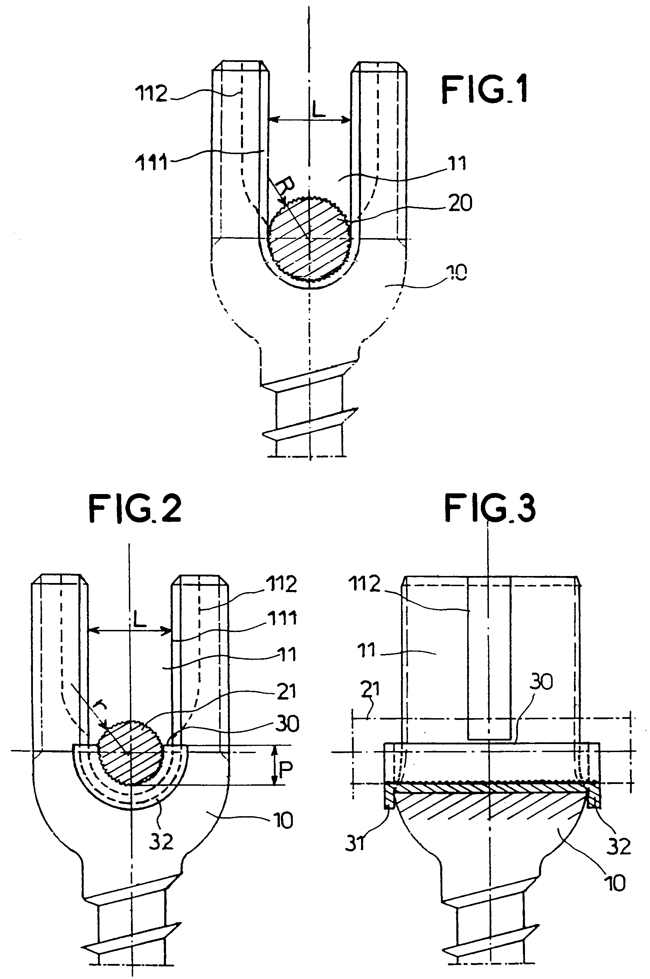

En examinant plus en détail les figures 1 à 3, on remarque qu'une tête d'implants à vis peut recevoir des tiges de différents diamètres **20**, **21**, sans que la qualité du blocage d'une tige **21** de diamètre inférieur à la largeur (L) de la rainure en soit altérée, sous réserve de l'interposition préalable, dans la rainure **11**, d'un adaptateur **30**, moleté intérieurement, à talons **31** et **32**, dont le rayon intérieur (r) correspond à celui de la tige **21** et dont la profondeur (P) est un peu supérieure au rayon intérieur (r) du dit adaptateur. En effet, par ce moyen, l'effet de sertissage de la dite tige **21**, par le rapprochement des côtés de la rainure **11**, sous l'effet de la conicité de l'écrou, subsiste, sans que le complément de blocage par l'écrou soit compromis. Cet exemple ne concerne que deux diamètres différents de tiges **20**, de rayon (R), et **21**, de rayon (r), mais rien ne s'oppose à ce qu'il soit étendu à d'autres dimensions, sous réserve que le rayon intérieur des adaptateurs soit déterminé en conséquence. Etant entendu que le même adaptateur **30** peut être utilisé pour relier des tiges de diamètre différent **20**, **21**, à l'aide des connecteurs **50** ou des plaquettes de liaison **65** à cavaliers **60**.

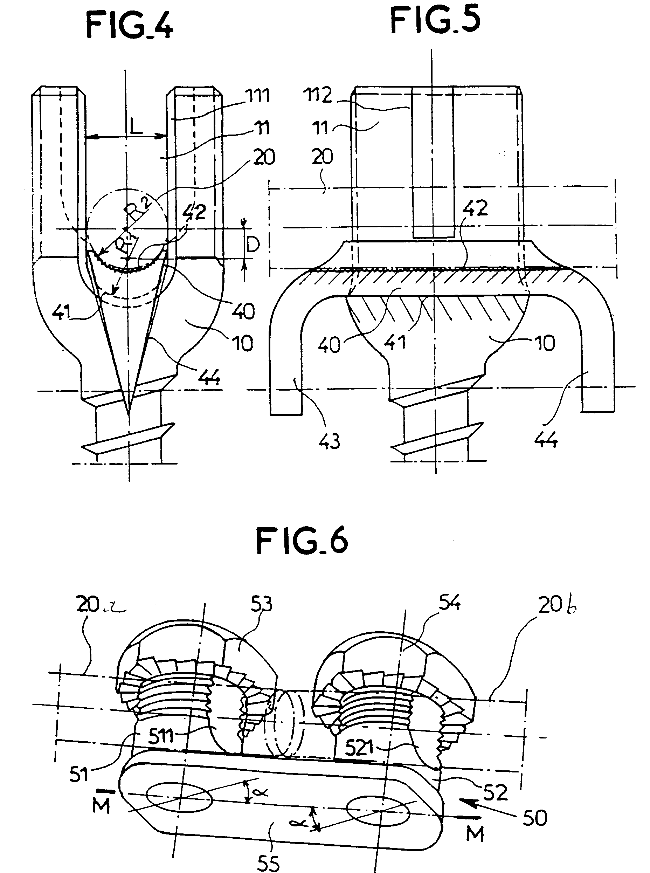

En examinant, maintenant, les figures 4 et 5, concernant l'agrafe **40**, on observe que, par ce moyen , les implants à vis sont solidarisés positivement en rotation par rapport aux vertèbres, indépendamment de la solidarisation en rotation de l'ensemble, par l'intermédiaire des tiges **20**, **21** qui les relient ; ceci par simple introduction, dans le fond de la rainure **11** des têtes de vis **10**, d'une agrafe **40**, après avoir dirigé les extrémités effilées **43** et **44** de celle-ci vers la vertèbre, avant d'introduire la tig**e 20** correspondante dans la rainure **11**. Ainsi, lors du blocage de la tige, sous l'effet conjugué du sertissage provoqué par le rapprochement des côtés de la rainure et du serrage par la base de l'écrou à filetage conique, les extrémités effilées **43**, **44** pénètrent dans la vertèbre, à'une profondeur suffisante pour obtenir une solidarisation totale en rotation de l'implant à vis par rapport à la vertèbre, compte-tenu, notamment, de la largeur des extrémités effilées **43**, **44** de l'agrafe **40** et de l'écartement de celles-ci ; ce qui a pour principal avantage d'éviter toute rotation de la vis par rapport à la vertèbre, s'opposant à la solidarisation en rotation naturelle par réaction de l'os par rapport au titane, dans lequel l'implant à vis est réalisé. A souligner que cette agrafe peut être mise à profit pour adapter la rainure **11** à une tige **21** de diamètre inférieur à la largeur (L) de la rainure, sous réserve que le rayon intérieur (R2) de l'agrafe soit réduit par rapport à son rayon extérieur (R1), avec un décalage (D) des axes de courbure, et que la profondeur de la dite agrafe soit déterminée de façon à être un peu supérieure à son rayon intérieur (R2).

En examinant la figure 6, on remarque que, en utilisant le connecteur **50** pour relier longitudinalement les éléments de tiges **20a** et **20b**, ceux-ci se trouvent réunis et solidarisés avec un certain désalignement latéral, compte-tenu du décalage angulaire alpha par rapport au plan médian longitudinal (MM) de la barrette **55**, introduite dans l'orientation des rainures **511** et **521** des têtes **51** et **52** ; ce désalignement des tiges **20a** et **20b** favorisant le libre réglage longitudinal de celles-ci, ainsi que leur fixation ; la rainure **511** ou **521** de l'une des têtes **51**, **52** pouvant être, à volonté, équipée d'un adaptateur **30**, pour permettre la liaison longitudinale de deux tiges **20**, **21** de diamètre différent.

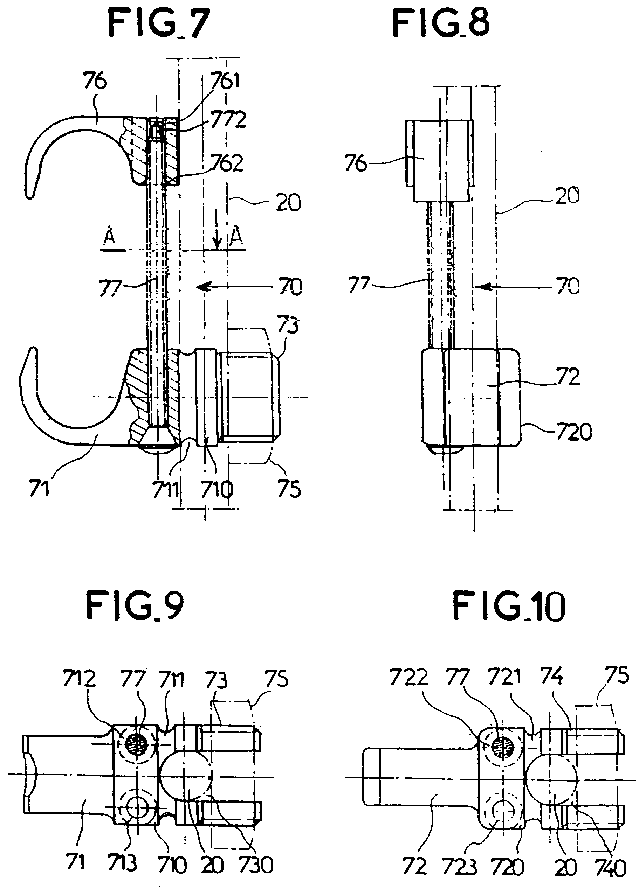

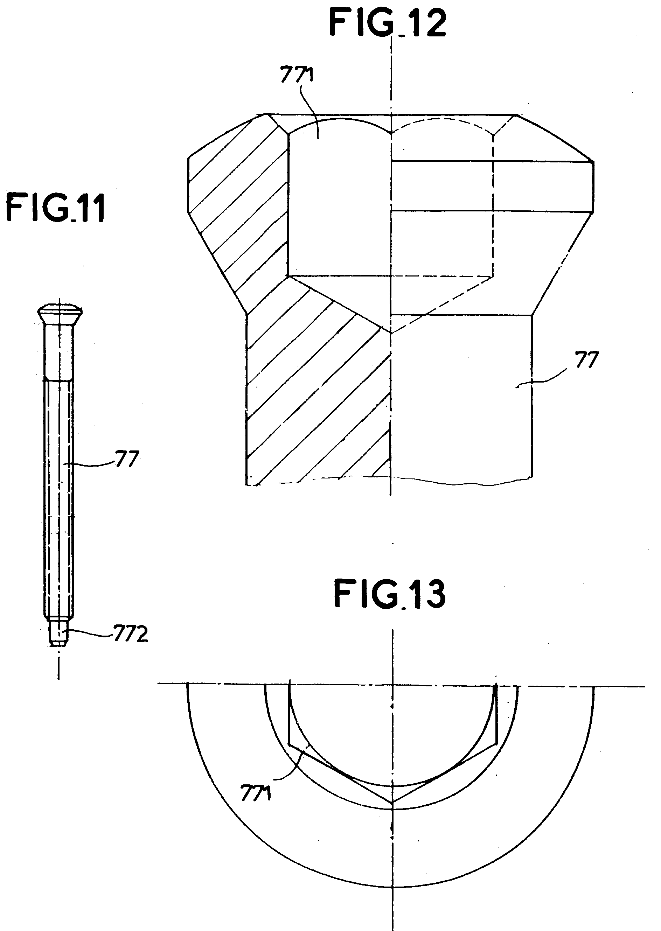

En se reportant, maintenant, aux figures 7 à 13, on constate qu'en utilisant les pinces pédiculaires ou laminaires **70**, les pédicules, ou lames, des vertèbres pourront être, tout d'abord, solidarisés à une tête **73** ou **74** rainurée **730** ou **740** à écrou de blocage **75**, par l'intermédiaire d'un crochet **71** ou **72** solidaire de la tête **73** ou **74** rainurée **730** ou **740** et d'un crochet simple **76**, indépendant, relié au premier **71** ou **72** par l'intermédiaire d'une vis à tête fraisée **77**, introduite, selon le cas, dans l'orifice **712** ou **713** de l'embase **710** du crochet pédiculaire **71**, ou **722** ou **723** du crochet laminaire **72**, puis vissée dans l'orifice fileté **761**, réalisé dans l'embase du crochet simple **76**, en recherchant l'alignement par action conjuguée de l'embout **772** de la vis **77** et de l'entrée conique **762** du filetage. Le blocage de la pince sur le pédicule, ou la lame, selon le cas, s'obtient à l'aide d'une clé à embout hexagonal, de dimensions appropriées à l'empreinte **771** de même forme, réalisée dans la tête hexagonale de la vis ; ce qui permet d'obtenir un serrage efficace, en utilisant, par exemple, une clé coudée, au lieu d'un tournevis. Cette fixation étant réalisée, il suffit, alors, de relier la tête **73** ou **74** du crochet **71** ou **72** à la tige **20**, par le moyen habituel, c'est-à-dire l'écrou **75**. Ainsi, en choisissant l'un ou l'autre des orifices **712** ou **713**, d'une part, ou **722** ou **723**, d'autre part, il est possible de positionner la pince d'un côté ou de l'autre de la tige concernée, tout en facilitant l'accès à la tête de la vis **77**, pour assurer sa manoeuvre, dans le cas où un réglage latéral est nécessaire.

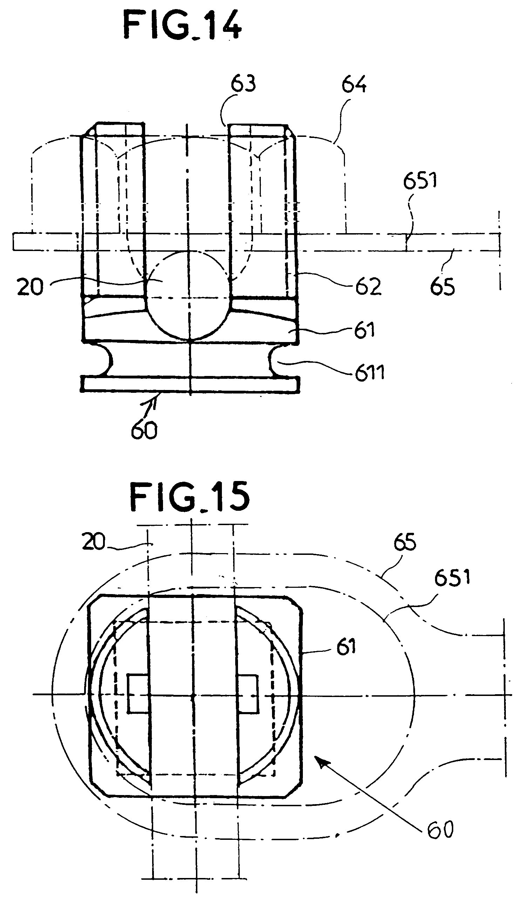

Les figures 14 et 15 montrent la façon dont deux tiges **20**, disposées parallèlement, peuvent être solidarisées latéralement, en utilisant une plaquette de liaison **65** à orifices de passage oblong **651**, deux cavaliers **60** à embase **61** à gorges **611** et à rainure **63** et deux écrous **64**.

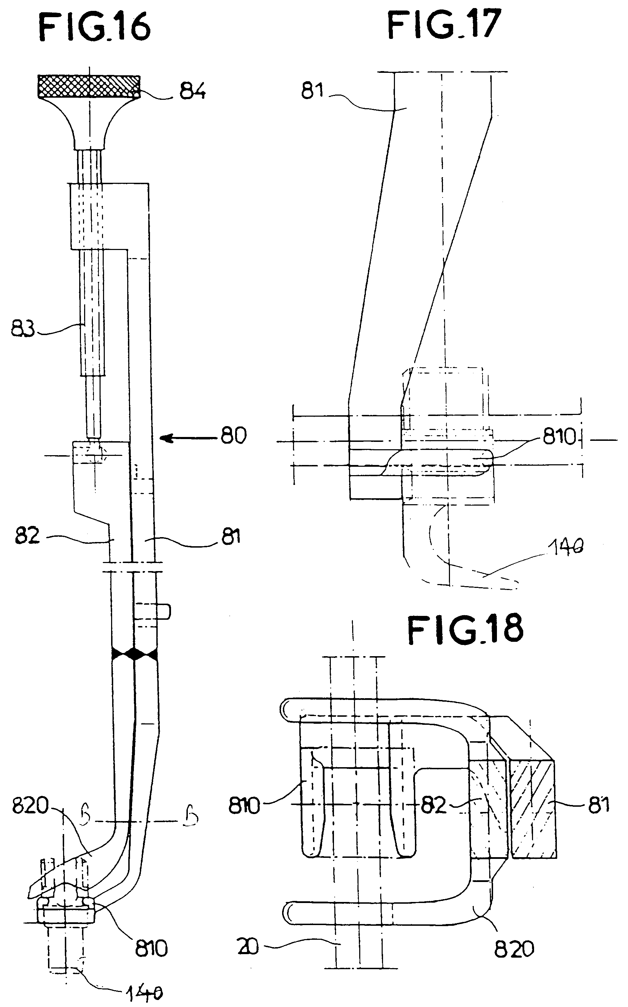

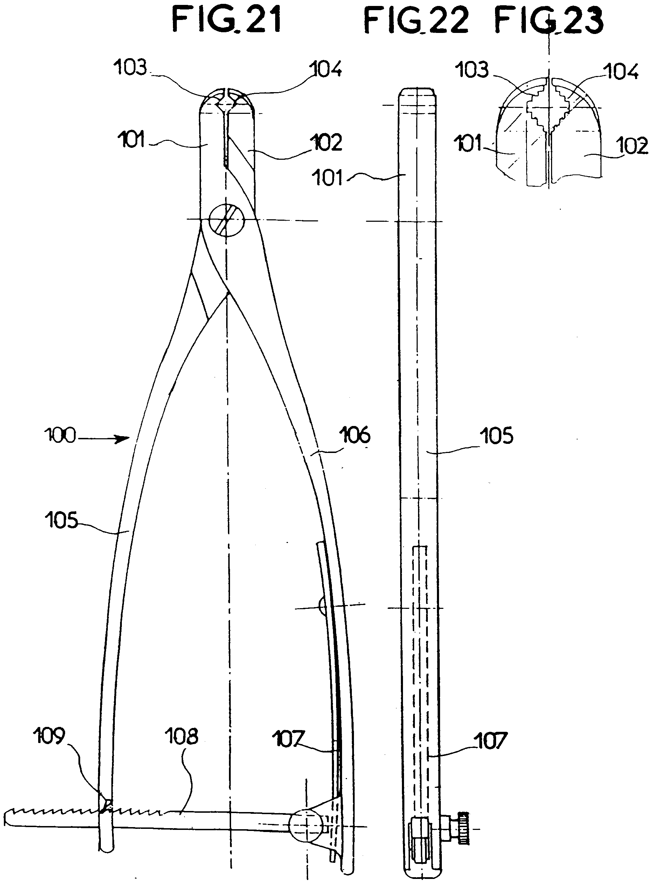

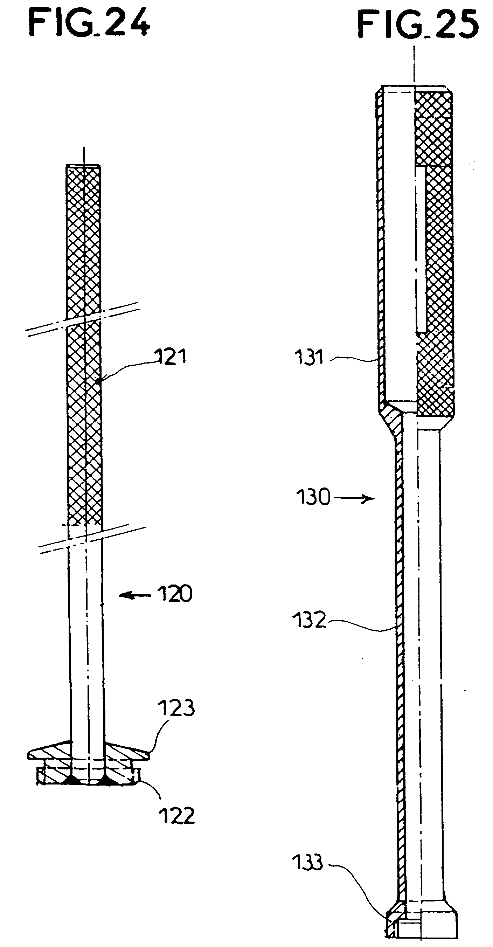

Les figures 16 à 18 montrent le pousse-tiges **80** permettant d'obtenir la mise en contact des tiges **20** avec le fond de rainure de la tête **62** des cavaliers **60**, ou **73** et **74** des crochets **71** et **72**, par introduction préalable des dents de la fourche transversale **810**, située à l'extrémité de la glissière **81** du pousse-tiges **80**, dans les gorges **611**, **711** ou **721** des dits cavaliers **60** et crochets **71** et **72**, puis flexion de la tige **20** sous l'action des dents de la fourche longitudinale **820** du coulisseau **82**, déplacé par rapport à la glissière **81** par l'intermédiaire de la vis **83** à bouton moleté **84**. L'espace correspondant ayant été laissé entre les dents de la fourche **820** pour permettre la mise en place et la libre rotation des écrous **64** ou **75**, par l'intermédiaire de l'outil représenté à la figure 24, qui pourra être, ensuite, retiré en même temps que le pousse-tiges **80**, pour laisser place à la clé, qui permettra d'assurer le blocage définitif, après que les tiges auront été tournées de 180°, à l'aide de la pince étau **100**, représentée aux figures 21 à 23, pour permettre le redressement du rachis ; afin d'obtenir une bonne solidarisation temporaire de la pince étau **100** à la tige, les becs **101**, **102** de la tige ont été munis d'empreintes en V **103**, **104**, striées transversalement, et le serrage est maintenu par une crémaillère **108** et un ergot **109**.

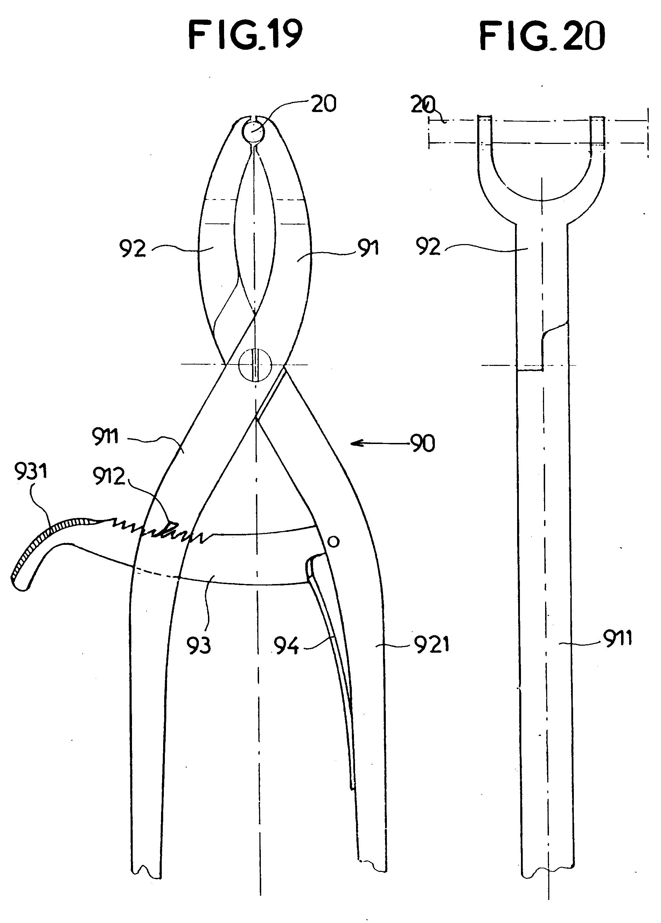

Les figures 19 et 20 représentent la pince anti-rotation **90**, utilisable pour positionner les tiges **20**, **21**, en tenant compte de leur déformation préalable exigée par la situation.

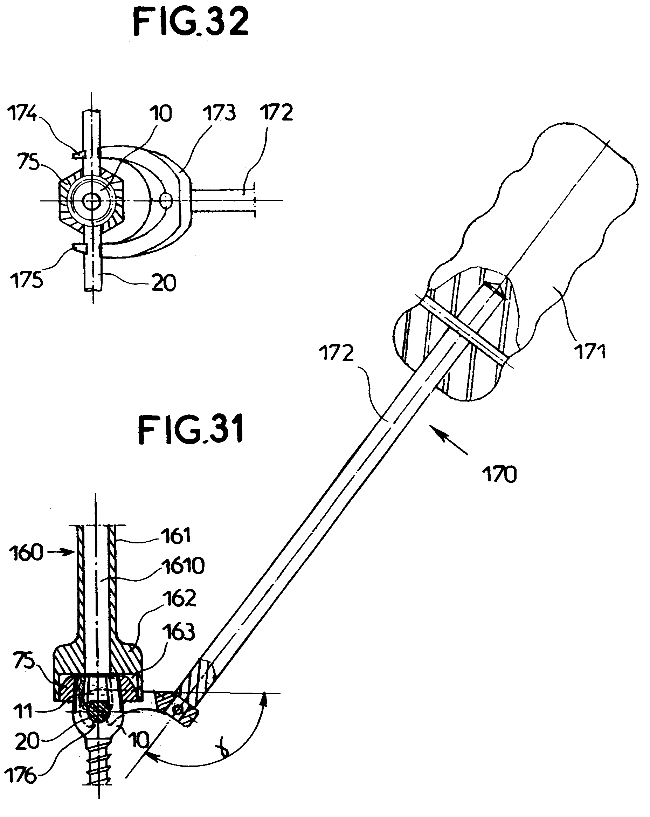

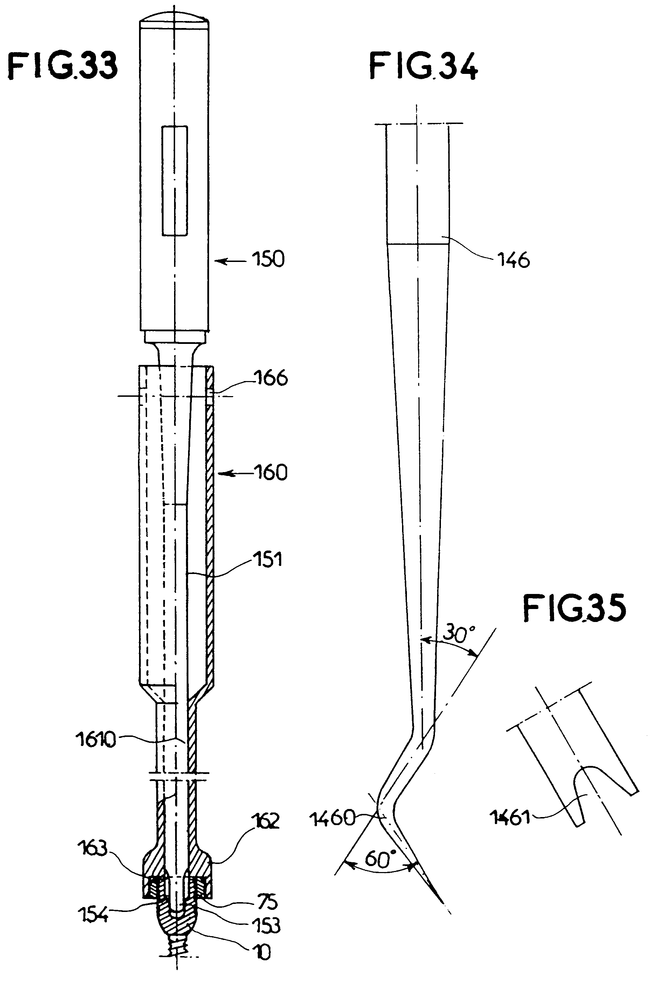

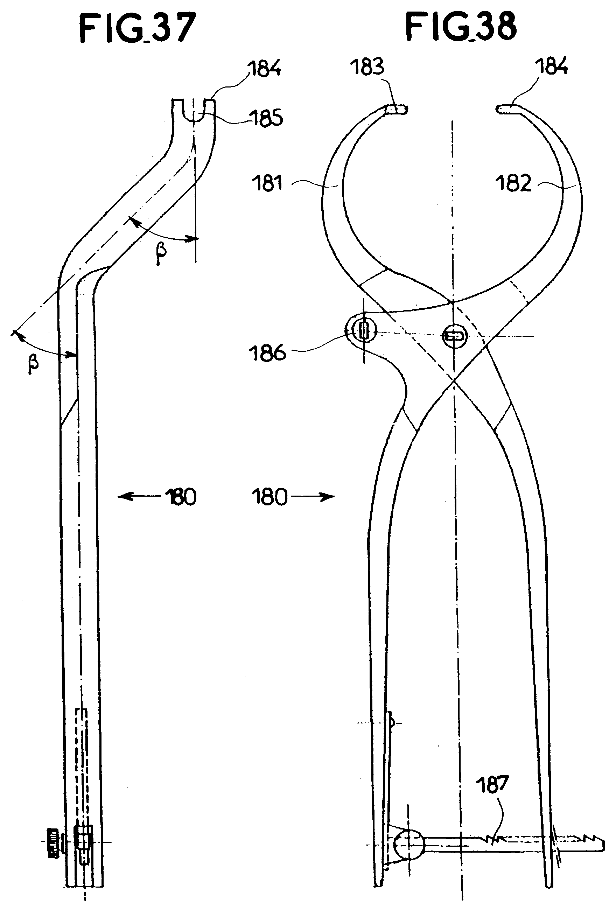

La pince étau **100** correspondant aux figures 21, 22, 23 est destinée à permettre la rotation des tiges **20**, **21**, avant rapprochement, ou écartement, des implants à vis, à l'aide d'une pince **180 ou 190** représentée aux figures 37 à 40 ; les dits implants étant maintenus en position par l'intermédiaire des mêmes pinces **180 ou 190**, grâce à leur crémaillère de blocage **187 ou 197**. La forme contrecoudée selon un angle bêta de 45° de l'extrémité utile des branches **181**, **182** ou **191**, **192** de ces pinces se prêtant au libre passage de la clé de serrage **160**, la solidarisation des implants, par rapport à une tige **20 ou 21**, peut être ainsi obtenue, puis le blocage définitif peut être assuré, en s'opposant à toute giration latérale de la tige à l'aide de la clé de contre-blocage, représentée figures 31 et 32, qui vient s'agripper sur la tige, de chaque côté de la tête de l'implant ; le couple de serrage de l'écrou pouvant être ainsi encaissé par la dite clé, grâce à la longueur de sa tige **172**.

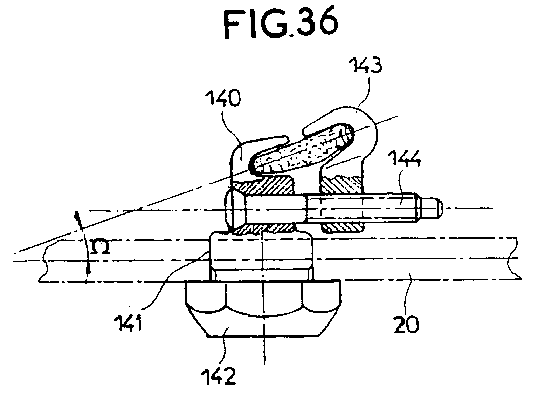

En se rapportant à la figure 36, représentant la pince auto-stable pour vertèbres cervicales, on remarque que les becs des crochets **140**, **143** sont, respectivement, relevés et rentrés selon un angle oméga d'environ 20°, de façon à tenir compte de la particularité de forme de ces vertèbres et à obtenir un auto-centrage lors du serrage avec la vis **144**; le montage et blocage en position de cette pince sur sa tige de liaison **21**, par l'intermédiaire de la tête rainurée **141** et de l'écrou **142**, pouvant être obtenus en utilisant les mêmes moyens que pour les implants à vis, les connecteurs de barres et les cavaliers des plaquettes de liaison, étant entendu qu'avant toute mise en place sur les pédicules, celles-ci ont été, au préalable, dégagées à l'aide du ciseau **146**, dont l'extrémité **1460**, contrecoudée à 30° et à 60°, comporte une partie tranchante, munie d'une échancrure en V arrondie **1461**.

Les figures 37 et 38 représentent une pince de compression **180** à branches **181**,**182** à butée d'ouverture **186**, à becs **183**,**184**, à échancrure **185** au diamètre de l'embase de la vis des implants **10**, avec blocage en position par une crémaillère escamotable **187**, permettant de rapprocher les implants **10** avant leur blocage sur une tige ou barre de liaison.

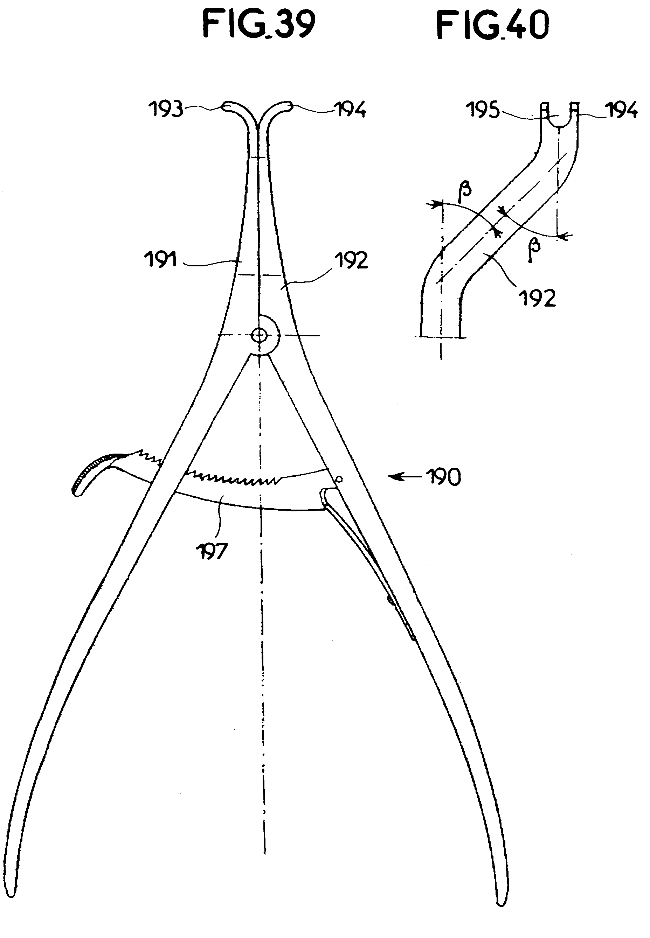

Les figures 39 et 40 représentent une pince de distraction **190** à branches **191**, **192** à becs **193**,**194** à échancrure **195** au diamètre de l'embase de la vis des implants **10** et à crémaillère de blocage **197**, permettant d'écarter les implants **10** avant leur blocage sur une tige ou une barre de liaison.

Les branches **181**,**182** et **191**,**192** des pinces de compression et de distraction représentées aux figures 37,38 et 39,40 sont coudées latéralement selon un angle bêta de 45° pour permettre le passage et la manoeuvre de la clé de serrage **160** des écrous **75**.

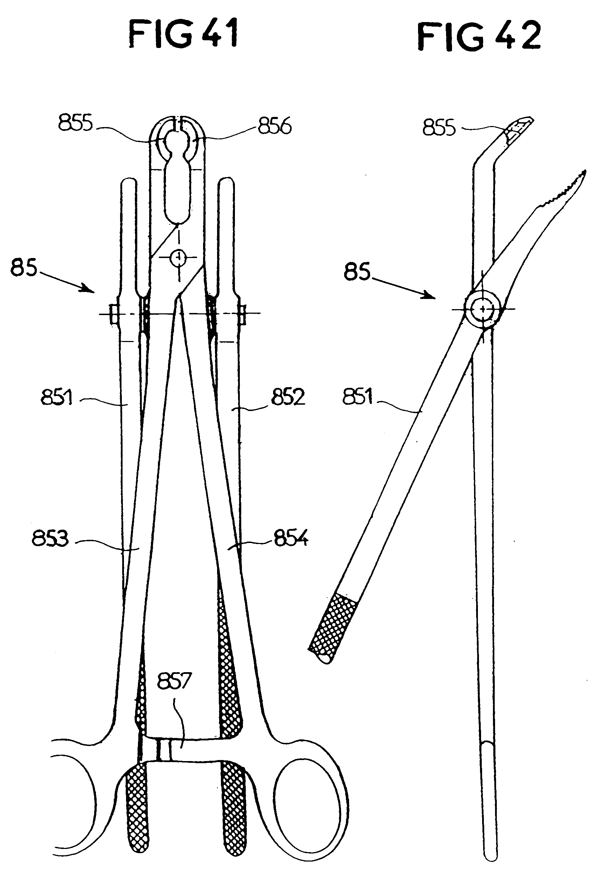

Les figures 41 et 42 représentent une pince pousse-tiges ou barres **85** constituée de deux leviers parallèles **851** et **852** à poignée moletée et à becs incurvés et striés destinés à prendre appui sur les tiges ou barres de chaque côté de la tête d'un implant **10**, articulés transversalement par rapport aux branches **853** et **854** d'une pince dont les becs **855** et **856** épousent la forme de la base sphérique de la tête des implants **10** ; les deux branches **853** et **854** pouvant, après rapprochement, être provisoirement solidarisées entre elles par l'intermédiaire d'une double crémaillère **857** après mise en place des becs **855** et **856** sous la base des implants **10**.

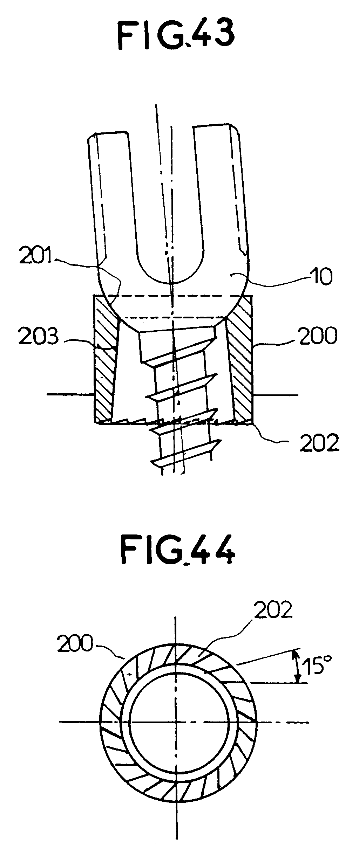

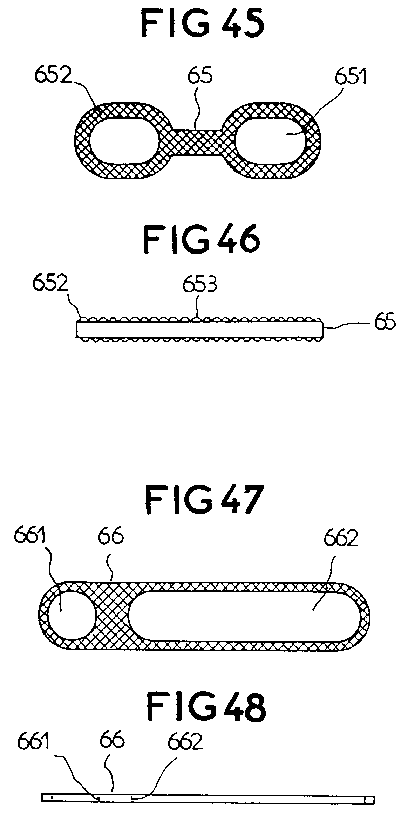

Les figures 43 et 44 représentent un implant à vis **10** muni d'une cale **200** comportant à sa partie supérieure, une surface d'appui sphérique **201** et à embase munie de stries **202** écartées angulairement selon un angle de 15°. La surface d'appui sphérique **201** et l'embase striée **202** étant reliées par un alésage tronconique **203** d'une conicité d'environ 5° dont la grande base est située vers le bas, de façon à laisser à l'implant une certaine latitude d'inclinaison par rapport à une surface préalablement applanie et creusée à l'aide d'une fraise de diamètre correspondant à celui de la cale **200**. Cette cale **200** pouvant être réalisée de différente hauteur pour pouvoir compenser, outre une certaine inclinaison de l'implant fileté **10**, un certain décalage dans le plan vertical par rapport à la surface initialement fraisée ; ladite cale offrant alors l'avantage de bien soutenir la tête de l'implant afin d'éviter toute rupture intempestive sous l'effet de contraintes latérales.

Les figures 45 et 46 représentent une plaquette de liaison **65** comportant deux orifices oblongs **651** et dont les faces comportent un moletage **652** croisé à 90° à arêtes applaties **653**. Ces plaquettes de liaison sont réalisées en différentes longueurs.

Les figures 47 et 48 représentent un autre mode de réalisation de plaquette de liaison **66**, comportant un orifice circulaire **661** et une rainure **662** couvrant toutes les possibilités d'écartement de tiges ou de barres. Cette plaquette **66** comporte sur ses faces un moletage croisé analogue à celui que comporte la plaquette de liaison **65**, ci-dessus décrite.

Le dispositif selon l'invention est destiné, principalement, aux prothèses rachidiennes à implants et crochets à tête rainurée, filetée extérieurement, et à écrou a filetage conique, telles que décrites dans le brevet français FR-A-2 650 173, mais rien ne s'opposerait à son application à d'autres prothèses rachidiennes, mettant en oeuvre des implants à tête rainurée, sous réserve d'une adaptation au demeurant assez mineure, ne sortant pas du cadre de la présente invention.

## Revendications

1. Dispositif de redressement et d'étaiement d'un rachis, constitué d'implants (**10**) vissés ou de crochets reliés par au moins deux tiges ou barres (**20**, **21**) moletées, solidarisées entre elles par l'intermédiaire d'éléments de raccordement et de liaison ; les dites tiges ou barres étant introduites dans des rainures (**11**), prévues à cet effet dans le corps des implants ou des crochets, puis bloquées dans le fond des rainures (**11**) par l'intermédiaire d'un système à vis cylindrique et écrou à filetage conique (**75**) ; les éléments de raccordement longitudinal des tronçons de tiges ou barres étant constitués d'une plaquette, comportant, à chaque extrémité, une tête d'implants, sur laquelle se monte un écrou (**75**) qui assure la solidarisation de l'ensemble ; les éléments de liaison latérale des tiges ou barres étant constitués d'une plaquette (**65**), comportant à chaque extremité un orifice de passage du corps de la tête d'un implant constituant un cavalier, la solidarisation de l'ensemble s'obtenant par l'intermédiaire d'écrous à filetage conique (**75**) ; les moyens de fixation des tiges ou barres aux lames et aux pédicules des vertèbres etant constitués de pinces comportant deux crochets, rappelés l'un vers l'autre par une vis, dont l'un au moins se fixe à la tige ou à la barre par une vis rainurée et un écrou à filetage conique, caractérisé en ce qu'un adaptateur (**30**) à talons (**31**, **32**), moleté intérieurement, épousant la forme du fond de la rainure (**11**) de la tête des implants (**10**) ou des crochets (**71**) et ayant un rayon intérieur correspondant à celui des tiges ou barres (**20**, **21**), de diamètre inférieur à la largeur de ladite rainure (**11**), se monte dans le fond de celle-ci, en ce que des chanfreins (**111**) sont réalisés sur les arêtes de chacune des rainures (**11**) des têtes d'implants (**10**), des cavaliers (**60**) ou des crochets (**71**, **140**), en ce que des rainures transversales (**112**) sont réalisées perpendiculairement à la rainure longitudinale (**11**) de la tête (**10**) des implants, de la tête des crochets (**71**, **140**) et de la tête des cavaliers des plaquettes de liaison (**65**, **66**), en ce que des agrafes (**40**), épousant la forme du fond de la rainure (**11**) de la tête des implants et celle des tiges ou barres (**20**, **21**), dont les extrémités effilées (**43**, **44**) sont pliées à 90° dans le sens de la vis d'implantation (**10**), se montent dans le fond de la rainure (**11**) de la tête des implants, en ce que les éléments de raccordement longitudinal des tronçons de tiges ou de barres sont constitués de connecteurs (**50**), comportant deux têtes d'implants (**51**, **52**), fixés aux extrémités d'une barrette (**55**), en ce que l'un au moins des orifices de passage (**651**) de la tête d'un cavalier (**60**), réalisés dans les plaquettes (**65**, **66**) de liaison latérale des tiges ou barres, est oblong, les dites plaquettes de liaison (**65**, **66**) comportant, sur leurs faces, un moletage (**652**) croisé à 90° et à arêtes aplaties (**653**), en ce que les pinces (**70**) de fixation des tiges ou barres aux lames ou aux pédicules des vertèbres dorsales sont constituées d'un crochet (**71**, **72**) à embase carrée (**710**, **720**), à gorges de préhension (**711**, **721**) et à tête (**73**, **74**) rainurée (**730**, **740**), d'un crochet simple (**76**), relié par une vis cylindrique à tête fraisée (**77**), à empreinte polygonale (**771**) et à embout d'alignement (**772**) venant se visser dans un orifice fileté (**761**), à ouverture conique (**762**), situé dans l'embase et dans le plan de symétrie du crochet simple (**76**), après passage par l'un des deux orifices lisses (**712**, **713**-**722**, **723**) réalisés dans l'embase (**710**, **720**) du crochet (**71**, **72**), parallèlement entre eux, dans un plan perpendiculaire au plan de symétrie dudit crochet (**71**, **72**), au voisinage immédiat de deux pans opposés de l'embase carrée (**710**, **720**), en ce que les pinces de fixation des tiges ou barres aux pédicules des vertèbres cervicales comportent un crochet (**140**) à tête rainurée (**141**) et à fixation par un écrou (**142**) et un crochet simple (**143**) dont les becs sont respectivement relevés et rentrés selon un angle oméga d'environ 20° par rapport à la tige ou à la barre, et en ce que l'inclinaison des implants à vis (**10**) par rapport aux vertèbres est compensée par des cales (**200**).

2. Dispositif selon la revendication 1, caractérisé en ce que les adaptateurs (**30**), destinés à se monter dans le fond des rainures (**11**) de tête (**10**) d'implants et de crochets les plus larges, en vue de permettre le montage d'une tige, ou barre, de diamètre inférieur à la largeur (**L**) de la rainure (**11**), ont une profondeur (**P**) un peu supérieure à leur rayon intérieur (**r**) et sont moletés intérieurement.

3. Dispositif selon la revendication 1, caractérisé en ce que les agrafes (**40**) comportent une partie convexe (**41**), de forme semi-circulaire correspondant à celle du fond de la rainure (**11**) de la tête (**10**) des implants, et une partie concave (**42**), moletée, de rayon (**R2**) correspondant à celui de la tige, ou barre (**20**, **21**), avec un décalage (**D**) des axes de courbure.

4. Dispositif selon la revendication 1, caractérisé en ce que les cales (**200**) pour les têtes d'implants à vis (**10**) comportent, à leur partie supérieure, une surface d'appui sphérique (**201**) et, à leur partie inférieure, une embase striée (**202**) ; la surface d'appui sphérique (**201**) et l'embase striée (**202**) étant reliées par un alésage tronconique (**203**), dont la grande base est située vers le bas.

5. Dispositif selon la revendication 1, caractérisé en ce que les cavaliers (**60**) de fixation des plaquettes de liaison latérale (**65**, **66**) sur les tiges ou barres sont rainurés longitudinalement et filetés extérieurement et comportent une embase carrée (**61**) munie d'une gorge de préhension (**611**).

6. Instrumentation de mise en oeuvre du dispositif selon les revendications 1 à 5, comprenant :
- un pousse-tiges, ou barres, (**80**), constitué d'une glissière (**81**), dont l'une des extrémités comporte une fourche transversale (**810**), dont les deux dents épousent la forme et l'écartement de gorges (**611**, **711** ou **721**) réalisées dans l'embase des crochets (**71** ou **72**) et des cavaliers (**60**), et d'un coulisseau (**82**) comportant, à l'une de ses extrémités, située du même côté que la fourche (**810**) de la glissière (**81**), une fourche longitudinale (**820**), à dents parallèles et incurvées, située au-dessus de la fourche (**810**) de la glissière ; la glissière (**81**) et le coulisseau (**82**) étant reliés par un système écrou et vis (**83**), à bouton moleté (**84**), assurant leur déplacement relatif,
- une pince anti-rotation (**90**) de tiges ou de barres (**20**, **21**), constituée de deux becs fourchus (**91**, **92**), comportant, à leur extrémité, des empreintes circulaires, de rayon correspondant à celui des tiges et des barres, rappelés l'un vers l'autre par des branches articulées (**911**, **921**), maintenues en position de serrage par une crémaillère (**93**) articulée par rapport à l'une des branches (**921**) rappelée par un ressort (**94**) vers un ergot (**912**), porté par l'autre branche (**911**) ; une gâchette de désengagement (**931**) étant située à l'extrémité de la crémaillère (**93**),
- une pince étau (**100**) porte-tiges, ou barres, constituée de deux becs (**101**, **102**), à empreintes en V (**103**, **104**) striées transversalement, rappelés l'un vers l'autre par des branches (**105**, **106**), maintenus en position de serrage par une crémaillère articulée (**108**), rappelée par un ressort (**107**) vers un ergot (**109**) porté par l'autre branche (**106**),
- un porte-écrou (**120**), constitué d'une tige (**121**), moletée sur une partie de sa longueur, comportant, à l'autre extrémité, un embout mâle fileté (**122**) à embase (**123**),
- un porte-vis et crochets (**130**), constitué d'un manche tubulaire (**131**) moleté, prolongé par une tige tubulaire (**132**) à embout femelle (**133**), fileté aux dimensions de la tête des implants à vis et des crochets,
- un tournevis (**150**) à ailettes d'entraînement (**153**, **154**), de section correspondant à celle des rainures longitudinale (**11**) et transversale (**112**), réalisées dans la tête des implants, des crochets, des connecteurs de barres et des cavaliers,
- une clé de serrage (**160**) à âme évidée (**1610**) selon le diamètre de la tige (**151**) du tournevis (**150**), comportant un pommeau démontable (**164**), relié à la poignée du dit tournevis (**150**) par l'intermédiaire d'un embout (**1641**), traversé par une tige de manoeuvre (**165**), passant par des orifices (**166**) percés transversalement dans la paroi du manche,
- une clé de contre-blocage (**170**), constituée d'une tige (**172**), munie, à une extrémité, d'une poignée (**171**), et, à l'autre extrémité, d'une fourche (**173**), inclinée selon un angle gamma de 135° par rapport à l'axe de la tige (**172**), dont l'extrémité des dents (**174**, **175**) comporte une échancrure semi-circulaire (**176**), de diamètre correspondant à celui de la tige de liaison (**20**, **21**),
- une pince de compression (**180**), constituée de deux branches articulées (**181**, **182**), à extrémité recourbée vers l'intérieur et contrecoudée selon un angle bêta de 45°, dans un plan perpendiculaire au plan d'articulation des branches, dont les becs (**183**, **184**) sont échancrés aux dimensions de l'embase de la vis d'implantation et sont opposés l'un à l'autre,
- une pince de distraction (**190**), constituée de deux branches articulées (**191**, **192**), à extrémités jointives et contrecoudées selon des angles bêta de 45°, dans un plan perpendiculaire au plan d'articulation des branches, comportant des becs (**193**, **194**) pliés à 90° dans des directions opposées et échancrés aux dimensions de l'embase de la vis d'implantation,
- un ciseau de préparation pédiculaire (**146**), comportant une extrémité contrecoudée à 30° et à 60°, dont l'extrémité coupante est munie d'une découpe en V arrondie (**1461**).
- une pince pousse-tiges ou barres (**85**) constituée de deux leviers (**851**, **852**) à becs incurvés et striés, articulés transversalement par rapport aux branches (**853 et 854**) d'une pince dont les becs (**855 et 85**6) épousent la forme de la base de la tête des implants à vis (**10**).

7. Instrumentation selon la revendication 6, caractérisée en ce que l'écartement des dents (**174**, **175**) de la fourche de la clé de contre-blocage est supérieur au diamètre de l'embout de la clé de serrage (**160**).

8. Instrumentation selon la revendication 6, caractérisée en ce que les becs (**183**, **184**) de la pince de compression (**180**) peuvent être maintenus en pression par l'intermédiaire d'une crémaillère escamotable (**187**), à coulissement limité par une butée (**186**) portée par l'une des branches (**182**).

9. Instrumentation selon la revendication 6, caractérisée en ce que les becs (**193**, **194**) de la pince de distraction (**190**) peuvent être maintenus écartés par l'intermédiaire d'une crémaillère escamotable (**197**).

10. Instrumentation selon la revendication 6, caractérisé en ce que les branches (**853**, **854**) de la pince pousse-tiges ou barre (**85**) peuvent être solidarisées en position fermée par l'intermédiaire d'une double crémaillère (**857**).

## Claims

1. Device for straightening and shoring a vertebral column comprising screwed implants (10) or hooks joined by at least two knurled rods or bars (20, 21) connected to each other by means of connecting and joining elements; said rods or bars being introduced into grooves (11) provided for this purpose in the body of the implants or hooks and then blocked in the base of the grooves (11) by a system consisting of a cylindrical screw and a conical threaded nut (75); the longitudinal connecting elements of the rod or bar sections being formed of a plate having at each end an implant head on which a nut (75) is mounted for joining the system; the lateral connecting elements of the rods and bars being formed of a plate (65) having at each end an opening for the body of an implant head forming a carrier, connection of the system being achieved by means of conical threaded nuts (75); the fixing means of the rods and bars to the blades and pedicles of the vertebrae being formed of clamps with two hooks, closed against each other by an screw, at least one of which is attached to the rod or to the bar by a grooved screw and a conical threaded nut, characterised in that an adaptor (30) with lugs (31, 32), knurled on the inside, which takes up the shape of the base of the groove (11) of the implants (10) or the hooks (71) and which has an internal radius corresponding to that of the rods or bars (20, 21), a diameter less than the width of said groove (11) and mounted in the bottom thereof, in that the chamfers (111) are connected to the stops of each groove (11) of the implant heads (10), of the carrier (60) or the hooks (71, 140), in that the transverse grooves (112) are connected perpendicularly to the longitudinal groove (11) of the implant head (10), the head of the hooks (71, 140) and the head of the carriers of the connecting plates (65, 66), in that the clamps (40) in the shape of the base of the groove (11) of the implant head and of the rods and bars (20, 21), the tapered ends of which (43, 44) are bent 90° in the direction of the implanting screw (10) and rise from the base of the groove (11) of the implant head, in that the longitudinal connecting elements of the rod or bar sections are made of connectors (50) having two implant heads (51, 52) attached to the end of a small bar (55), in that at least one of the passage openings (651) of the carrier head (60) made in the connecting plates (65, 66) of the lateral rod or bar connection is oblong, said connecting plates (65, 66) having on their face side knurling (652) crossed at 90° and flattened stops (653), in that the clamps (70) for fixing the rods or bars to the blades or pedicles of dorsal vertebrae comprise a hook (71, 72) with a square base (710, 720), clamping grooves (711, 721) and a grooved (730, 740) head (73, 74), a simple hook (76) connected by a cylindrical screw with a countersunk head (77), with a polygonal recess (771) and an alignment joint (772) which screws into a threaded opening (761) with a conical aperture (762) located in the base and in the plane of symmetry of the simple hook (76) after passing through one of the two smooth openings (712, 713-722, 723) in the base (710, 720) of the hook (71, 72) parallel between them perpendicularly to the plane of symmetry of said hook (71, 72) in the immediate vicinity of the two opposite faces of the square base (710, 720) and in that the clamps for fixing the rods or bars to the pedicles of the cervical vertebrae have a hook (140) with a grooved head (141) and for fixing by a nut (142) and a simple hook (143) the points of which are respectively opened and closed in accordance with an omega angle of approx. 20° with regard to the rod or the bar, and in that the inclination of the screw implants (10) with regard to the vertebrae is compensated by blocks (200).

2. Device according to claim 1, characterised in that the adaptors (30) for mounting in the base of the grooves (11) of the head (10) of the implants and larger hooks in order to permit the assembly of a rod or bar with a diameter smaller than the width (L) of the groove (11), have a depth (P) a little larger than the internal radius (r) and are internally knurled.

3. Device according to claim 1, characterised in that the clamps (40) have a convex section (41) of semi-circular shape which corresponds to that of the base of the groove (11) of the head (10) of the implants, and a concave knurled section (42) with a radius (R2) corresponding to that of the rod or bar (20, 21) with displacement (D) of the curve axes.

4. Device according to claim 1, characterised in that the blocks (200) for the screw implant heads (10) have on their upper section, a spherical support surface (201) and on their lower section a grooved base (202); the spherical support surface (201) and the grooved base (202) being connected by a truncated hole (203) the large base of which is towards the bottom.

5. Device according to claim 1, characterised in that the carriers (60) for fixing the lateral connecting plates (65, 66) to the rods or bars are longitudinally grooved and are externally threaded and have a square base (61) with a gripping groove (611).

6. Instrument for using the device in accordance with claims 1 to 5 comprising:
- a rod or bar push (80) formed of a slide rail (81) one of the ends of which has a transverse fork (810) the two teeth of which are of the shape and spacing of the grooves (611, 711 or 721) in the base of the hooks (71 or 72) and the carrier (60), and a slide block (82) having at one of its ends located on the same side as the fork (810) of the slide rail (81), a longitudinal fork (820) with parallel and inward-bent teeth located above the fork (810) of the slide rail; the slide rail (81) and the slide block (82) being connected by a screw and nut system (83) with a knurled button (84) which ensures their movement relative to each other;
- anti-rotation pliers (90) for the rods or bars (20, 21) formed of two forked jaws (91, 92) having at their ends circular indentations with a radius corresponding to that of the rods or bars, closed against each other by hinged arms (911, 921) kept in the clamping position by a rack (93) which is hinged to one of the arms (921) and closed by means of a spring (94) to snug (912) on the other arm (911); a disengaging device (931) being located at the end of the rack (93);
- a vice grip (100) rod or bar holder comprising two jaws (101, 102) with V-shaped recesses (103, 104) which are transversely grooved and closed against each other by arms (105, 106) and held in the clamped position by a hinged rack (108) closed by a spring (107) against a snug (109) carried by the other arm (106);
- a nut holder (120) comprising a rod (121) knurled along part of its length and having at its other end a threaded male joining piece (122) at the base (123);
- a screw and hook holder (130) comprising a knurled tubular sleeve (131) extended by a tubular rod (132) with a female joining piece (133) which is threaded to the dimensions of the screw and hook implant heads;
- a screwdriver (150) with drive blades (153, 154) with a cross-section corresponding with that of the longitudinal (11) and transverse (112) grooves in the head of the implants, the hooks, the bar connectors and carriers;
- a closing key (160) with a hollow core (1610) in accordance with the diameter of the rod (151) of the screwdriver (150) having a detachable cover (164) connected to the handle of said screwdriver (150) by means of a connecting piece (1641) crossed by a manoeuvering rod (165) passing through openings (166) pierced transversely in the wall of the sleeve;
- an anti-blocking key (170) comprising a rod (172) having a handle (171) at one end and at the other end a fork (173) inclined at a gamma angle of 135° with regard to the axis of the rod (172), the ends of the teeth (174, 175) of which have a semi-circular scallop (176) of a diameter corresponding to that of the connecting rod (20, 21);
- compression pliers (180) comprising two hinged arms (181, 182) curved in at the ends towards the interior and offset at a beta angle of 45° perpendicular to the arm hinging plane, the jaws (183, 184) of which are scalloped to the dimensions of the base of the setting screw and are opposite each other;
- distraction pliers (190) comprising two hinged arms (191, 192) joined at the ends and offset by beta angles of 45° perpendicular to the hinging plane of the arms, having jaws (193, 194) bent 90° in opposite directions and scalloped to the dimension of the base of the setting screw;
- a pedicle preparation cutter (146) with an end bent at 30° and 60°, the cutting end of which has a rounded V-shaped slot (1461);
- rod or bar pushing pliers (85) comprising two levers (851, 852) which are curved inwards and grooved, hinged transversely with regard to the arms (853 and 854) of pliers the jaws (855 and 856) of which are in the shape of the base of the head of the screw implants (10).

7. Instrument in accordance with claim 6, characterised in that the spacing of the teeth (174, 175) of the fork of the anti-blocking key is greater than the diameter of the connecting piece of the closing key (160).

8. Instrument in accordance with claim 6, characterised in that the jaws (183, 184) of the compression pliers (180) can be kept under pressure by means of a retractable rack (187) with a slide limited by a stop (186) on one of the arms (182).

9. Instrument according to claim 6, characterised in that the jaws (193, 194) of the distraction pliers (190) can be kept apart by means of a retractable rack (197).

10. Instrument according to claim 6, characterised in that the arms (853, 854) of the rod or bar pushing pliers (85) can be locked into a closed position by means of a double rack (857).

## Patentansprüche

1. Vorrichtung zum Korrigieren und Stützen einer Wirbelsäule, umfassend ein verschraubtes Implantat (10) oder Haken, die mittels zweier gerändelter Stäbe oder Stangen (20, 21) verbunden sind, untereinander mittels Anschluß- und Verbindungselementen miteinander verbunden; die genannten Stäbe oder Stangen sind eingeführt in Nuten (11), die zu diesem Zwecke im Körper der Implantate oder der Haken vorgesehen sind, sodann am Grund der Nuten (11) mittels eines Systems zylindrischer Schrauben und Muttern mit konischem Gewinde (75) verriegelt sind; die längs sich erstreckenden Verbindungselemente von Stab- oder Stangenabschnitten bestehen aus einer Platte, die an jedem Ende einen Implantatkopf aufweist, an welchem eine Schraube (75) montiert ist, die den Zusammenhalt der Einheit sicherstellt; die seitlichen Verbindungselemente der Stäbe oder Stangen bestehen aus einer Platte (65), die an jedem Ende eine Durchgangsbohrung des Körpers des Kopfes eines Implantats aufweist und dabei einen Reiter bildet, wobei der Zusammenhalt der Einheit mittels konischer Schrauben (75) erzielt wird, wobei die Befestigungsmittel der Stäbe oder Stangen an den Plättchen und den Pediculen der Wirbel aus Klammern bestehen, welche Haken umfassen, die durch eine Schraube zusammengedrückt sind, und deren eine am Stab oder der Stange mittels einer genuteten Schraube und einer Mutter mit konischem Gewinde befestigt ist,
dadurch gekennzeichnet, daß ein mit Ansätzen (31, 32) versehener, innengerändelter Adapter (30), der die Gestalt des Bodens der Nut (11) des Kopfes des Implantats (10) oder der Haken (71) aufweist und einen Innendurchmesser hat, der jenem der Stäbe oder Stangen (20, 21) entspricht, von kleinerem Durchmesser als der Breite der genannten Nut (11), in deren Boden montiert ist, daß an den Stegen (111) einer jeden Nut (11) der Implantatköpfe (10), der Reiter (60) und der Haken (71, 140) Anfasungen vorgesehen sind, daß Quernuten (112) senkrecht zur Längsnut (11) des Kopfes (10) des Implantats, des Kopfes der Haken (71, 140) und des Kopfes der Reiter der Verbindungsplatten (65, 66) vorgesehen sind, daß Klammern (40), die sich der Gestalt des Bodens der Nut (11) des Kopfes des Implantats und jener der Stäbe oder Stangen (20, 21) anpassen, und deren spitz zulaufende Enden (43, 44) um 90° im Sinne der Implantatschraube (10) abgekröpft sind, in den Boden der Nut (11) des Implantatkopfes einmontiert sind, daß die in Längsrichtung verlaufenden Verbindungselemente der Abschnitte der Stäbe oder Stangen aus Anschlüssen (50) bestehen, umfassend zwei Implantatköpfe (51, 52), die an den Enden eines Jochs (55) fixiert sind, daß wenigstens eine der Durchgangsbohrungen (651) des Kopfes eines Reiters (60), vorgesehen in den seitlichen Verbindungsplatten (65, 66) der Stäbe oder Stangen, langgestreckt ist, daß die genannten Verbindungsplatten (65, 66) an ihren Seitenflächen eine Rändelung (652) aufweisen mit einer Überkreuzung von 90° und mit abgeplatteten Schnittkanten (653), daß die Klammern (70) zum Befestigen der Stäbe oder Stangen an den Plättchen oder Pediculen der Rückenwirbel aus einem Haken (71, 72) mit quadratischer Basis (710, 720) mit Haltenut (711, 721) und mit einem genuteten (730, 740) Kopf (73, 74) bestehen, einem einfachen Haken (76), der verbunden ist mittels einer zylindrischen Schraube mit gefrästem Kopf (77), mit einer polygonalen Aussparung (771) und mit einem Zentrierübergang (772), der in eine Gewindebohrung (761) einschraubbar ist, mit einer konischen Bohrung (762), die in der Basis und in einer Symmetrieebene des einfachen Hakens (76) angeordnet ist, nach Durchgang durch eine der beiden Bohrungen (712, 713-722, 723), die in der Basis (710, 720) des Hakens (71, 72) vorgesehen ist, die zueinander parallel verlaufen, in einer zur Symmetrieebene des genannten Hakens (71, 72) senkrechten Ebene in der unmittelbaren Nachbarschaft von zwei einander gegenüberliegenden Ebenen der quadratischen Basis (710, 720), daß die Befestigungsklammern der Stäbe oder Stangen an den Pediculen der Halswirbel einen Haken (140) mit genutetem Kopf (141) aufweisen sowie zur Befestigung mittels einer Schraube (142) und einen einfachen Haken (143), deren Öffnungen angehoben und gemäß einem Winkel Omega von etwa 20° in bezug auf den Stab oder die Stange geneigt sind, und daß die Neigung des Schraubenimplantats (10) in bezug auf die Wirbel kompensiert ist durch Keile (200).

2. Vorrichtung nach Anspruch 1, dadurch gekennzeichnet, daß die Adaptoren (30), die dazu vorgesehen sind, im Boden der Nuten (11) des Implantatkopfes (10) und der größten Haken montiert zu werden, eine Tiefe (P) aufweisen, die an ihrem inneren Radius (r) etwas größer ist, und im Inneren gerändelt sind, um das Montieren eines Stabes oder einer Stange von einem Durchmesser zu erlauben, der kleiner ist als die Breite (L) der Nut (11).

3. Vorrichtung nach Anspruch 1, dadurch gekennzeichnet, daß die Klammern (40) einen konvexen Teil (41) von halbkreisförmiger Gestalt aufweisen, entsprechend jenem des Bodens der Nut (11) des Implantatkopfes (10), sowie einen konkaven, gerändelten Teil (42) von einem Radius (R2) entsprechend jenem des Stabes oder der Stange (20, 21) bei einer Verschiebung (D) der Krümmungsachsen.

4. Vorrichtung nach Anspruch 1, dadurch gekennzeichnet, daß die Keile (200) für die mit Gewinde versehenen Implantatköpfe (10) an ihrem oberen Teil eine sphärische Stützfläche (201) und an ihrem unteren Teil eine gerillte Basis (202) aufweisen, und daß die sphärische Stützfläche (201) und die gerillte Basis (202) durch eine kegelstumpfförmige Bohrung (203) miteinander verbunden sind, deren großer Durchmesser sich unten befindet.

5. Vorrichtung nach Anspruch 1, dadurch gekennzeichnet, daß die Befestigungsreiter (60) der seitlichen Verbindungsplatten (65, 66) auf den Stäben oder Stangen in Längsrichtung genutet und außen mit Gewinde versehen sind und eine quadratische Basis (61) aufweisen, die eine Greifrille (611) aufweist.

6. Instrumentierung zum Gebrauch der Vorrichtung nach den Ansprüchen 1 bis 5, umfassend:
- einen Stab- oder Stangenschieber (80), bestehend aus einer Gleitführung (81), deren eines Ende eine Quergabel (810) aufweist, deren beide Zinken der Gestalt und der Spreizung von Rillen (611, 711 oder 721) angepaßt sind, die ihrerseits in der Basis von Haken (71 oder 72) und von Reitern (60) vorgesehen sind, und aus einem Gleitkörper (82), der an einem seiner beiden Enden, welches sich auf derselben Seite wie die Gabel (810) der Gleitführung (81) befindet, eine Längsgabel (820) aufweist mit zueinander parallelen, nach innen gekrümmten Zinken, angeordnet oberhalb der Gabel (810) der Gleitführung, wobei die Gleitführung (81) und der Gleitkörper (82) durch ein Schrauben- und Mutternsystem (83) mit einer gerändelten Mutter (84) miteinander verbunden sind, die deren Relativbewegung ermöglicht;
- eine Anti-Rotationszange (90) der Stäbe oder Stangen (20, 21), bestehend aus zwei Gabelklauen (91, 92), die an ihren Enden kreisförmige Aussparungen von einem Radius aufweisen, der jenem der Stäbe oder Stangen entspricht, und die gegeneinander gedrückt werden durch Gelenkhebel (911, 921), die ihrerseits in Spannposition gehalten werden durch eine Zahnstange (93), die in bezug auf einen der Gelenkhebel (921) durch eine Feder (94) gegen einen Anschlag (912) gedrückt ist, getragen vom anderen Hebel (911), wobei ein Auslöser (931) am Ende der Zahnstange (93) angeordnet ist;
- eine Klemmzange (100) für die Stangen oder Stäbe, umfassend zwei Klauen (101, 102) mit V-förmigen Aussparungen (103, 104), die quergerillt sind, zusammengedrückt durch Hebel (105, 106), die ihrerseits in Spannposition gehalten sind durch eine angelenkte Zahnstange (108), die durch eine Feder (107) gegen einen Anschlag (109) gedrückt sind, getragen vom anderen Hebel (106);
- einen Schraubenträger (120), bestehend aus einem Schaft (121), der auf einem Teil seiner Länge gerändelt ist und der an seinem anderen Ende eine Schraubzwinge (122) mit Basis (123) aufweist;
- einen Schrauben- und Hakenträger (130), bestehend aus einer gerändelten Hülse (131), die durch einen hülsenförmigen Schaft (132) mit innenverschraubter Zwinge (133) verlängert ist, mit Gewinde versehen entsprechend den Dimensionen des Implantatkopfes mit Schrauben und Haken;
- einen Schraubenzieher (150) mit Mitnehmerflügeln (153, 154) von einem Querschnitt entsprechend jenem der Längsnuten (11) und der Quernuten (112), vorgesehen im Kopf der Implantate, der Haken, der Anschlüsse der Stäbe und der Reiter;
- einen Spannschlüssel (160) mit einer Bohrung (1610) entsprechend dem Durchmesser des Schaftes (151) des Schraubenziehers (150), umfassend einen abnehmbaren Knauf (164), der an den Handgriff des Schraubenziehers (150) mittels einer Zwinge (1641) angeschlossen ist, durch die ein Handhabungsstab (165) hindurchgeführt ist, der durch Querbohrungen (166) in der Wand der Hülse verläuft;
- einen Gegenblockierschlüssel (170), umfassend einen Schaft (172), der an seinem einen Ende mit einem Handgriff (171) und an seinem anderen Ende mit einer Gabel (173) versehen ist, die ihrerseits unter einem Winkel Gamma von 135° in bezug auf die Achse des Schaftes (172) geneigt ist, und deren Zinken (174, 175) eine halbkreisförmige Aussparung (176) von einem Durchmesser bilden, der jenem des Verbindungsschaftes (20, 21) entspricht;
- eine Druckzange (180), umfassend zwei Gelenkhebel (181, 182) mit nach innen abgekröpften Enden und doppelt gekröpft entsprechend einem Winkel Beta von 45° in einer Ebene senkrecht zur Gelenkebene der Hebel, deren Greifteile (183, 184) entsprechend den Abmessungen der Basis der Implantatschraube geschweift und einander zugewandt sind;
- eine Distraktionszange (190), umfassend zwei Gelenkhebel (191, 192) mit dicht aneinander anliegenden Enden, die unter Winkeln Beta von 45° in einer zur Gelenkebene der Hebel senkrechten Ebene doppelt gekröpft sind, umffassend Greifteile (193, 194), die unter 90° in einander entgegengesetzten Richtungen umgebördelt und geschweift sind entsprechend den Dimensionen der Basis der Implantatschraube;
- einen pedicularen Präparationsmeisel (146) mit einem unter 30° und unter 60° doppelt gekröpften Ende, dessen Schneidende versehen ist mit einer Aussparung in der Gestalt eines gerundeten V (1461);
- eine Stab- oder Stangenschieberzange (85), umfassend zwei Hebel (851, 852) mit gekrümmten und gerändelten Griffteilen, die gekrümmt und gerändelt sind, quer angelenkt in bezug auf die Hebel (853 und 854) einer Zange, deren Greifteile (855 und 856) der Gestalt der Basis des Kopfes der Schraubenimplantate (10) angepaßt ist.

7. Instrumentierung nach Anspruch 6, dadurch gekennzeichnet, daß die Spreizung der Zinken (174, 175) der Gabel des Gegenblockierschlüssels größer ist als der Durchmesser der Zwinge des Spannschlüssels (160).

8. Instrumentierung nach Anspruch 6, dadurch gekennzeichnet, daß die Greifteile (183, 184) der Druckzange (180) mittels einer versenkbaren Zahnstange (187) unter Druck gehalten werden können, bei begrenzter Gleitbewegung durch einen Anschlag (186), der von einem der Hebel (182) getragen ist.

9. Instrumentierung nach Anspruch 6, dadurch gekennzeichnet, daß die Greifteile (193, 194) der Distraktionszange (190) mittels einer verfahrbaren Zahnstange (197) gespreizt gehalten werden können.

10. Instrumentierung nach Anspruch 6, dadurch gekennzeichnet, daß die Hebel (853, 854) der Stab- oder Stangenschieberzange (85) mittels einer doppelten Zahnstange (857) in ihrer geschlossenen Position festgesetzt werden können.
